# EUROPÄISCHE PATENTSCHRIFT

(11) **EP 3 717 107 B1**
(45) Veröffentlichungstag und Bekanntmachung des Hinweises auf die Patenterteilung: **30.08.2023**
(21) Anmeldenummer: 18788751.8
(22) Anmeldetag: 16.10.2018
(51) Int. Cl.: B01D 63/08, B01D 29/085, C12M 3/00, B01D 29/05, B01D 69/10

(54) **FILTRATIONSVORRICHTUNG FÜR VAKUUM-MEMBRANFILTRATION**
FILTRATION DEVICE FOR VACUUM MEMBRANE FILTRATION
APPAREIL DE FILTRATION DESTINÉE À UNE FILTRATION À MEMBRANE SOUS VIDE

(30) Priorität: 27.11.2017 DE 102017127970
(43) Veröffentlichungstag der Anmeldung: 07.10.2020
(73) Patentinhaber: Sartorius Stedim Biotech GmbH, 37079 Göttingen (DE)
(72) Erfinder: PRUEHL, Sebastian, 37079 Goettingen (DE); PFLANZ, Karl, 37079 Goettingen (DE); GROSSMANN, Juliane, 37079 Goettingen (DE); SCHUETZLER, Michael, 37079 Goettingen (DE)
(74) Vertreter: Novagraaf International SA
(86) Internationale Anmeldenummer: PCT/EP2018/078182
(87) Internationale Veröffentlichungsnummer: WO 2019/101445

(56) Entgegenhaltungen:
- EP-A2- 1 911 519
- DE-B3-102005 008 220
- US-A- 4 777 137
- US-A- 4 792 398
- US-A1- 2006 144 781

## Beschreibung

Die Erfindung betrifft eine Filtrationsbasis für Vakuum-Membranfiltrationsanwendungen.

Eine Filtrationsbasis bildet zusammen mit einem Aufgusstrichter und einem zwischen diesen beiden Komponenten geklemmten kreisförmigen Membranfilter eine Filtrationsvorrichtung, an die im Filtrationsbetrieb üblicherweise ein Vakuum (Unterdruck) angelegt wird. Bei bekannten Vorrichtungen wird die Klemmung des Membranfilters durch eine umlaufende Auswölbung am Fuße des Trichters und durch einen umlaufenden Rücksprung an der Filtrationsbasis sichergestellt. Die Auswölbung kann in den Rücksprung eingreifen, wodurch der Außenumfang des Membranfilters auf eine Dichtfläche der Filtrationsbasis gedrückt wird. In die Trichter solcher Filtrationsvorrichtungen können typischerweise Flüssigkeitsvolumen von 0 bis 300 ml eingefüllt werden. Im Filtrationsbetrieb wird an die Unterseite des Membranfilters ein Vakuum angelegt, um die im Trichter befindliche Flüssigkeit durch den auf der Filtrationsbasis aufliegenden Membranfilter anzusaugen. Hierfür besitzt die Filtrationsbasis an ihrer Unterseite eine Dichtkontur für den Anschluss an eine Absaugvorrichtung.

Nach der Filtration mit einer aus Filtrationsbasis, Membranfilter und Trichter gebildeten Filtrationsvorrichtung kann mit einer Vakuumpumpe aufgrund des hohen Blasendruckes keine Luft durch den Membranfilter gesaugt werden. Dies liegt daran, dass die Poren des Membranfilters so klein sind, dass das Vakuum die Oberflächenspannung des Wassers in diesen kleinen Poren nicht überwinden kann. Außerdem liegt der Membranfilter auf der Filtrationsbasis auf, zu der er nach der Benetzung mit Wasser auch nach der Entfernung des Trichters eine dichte Verbindung aufweist. Aus diesen Gründen befindet sich nach einer Filtration noch eine beachtliche, nicht ohne Weiteres absaugbare Flüssigkeitsmenge unter dem Membranfilter, was insbesondere bei mikrobiologischen Arbeiten zu Problemen führen kann. Eine Filtrationsvorrichtung mit einer Filtrationsbasis ist aus DE 10 2005 008220 B3 bekannt.

Aufgabe der Erfindung ist es daher, bei einer Vakuum-Membranfiltrationsvorrichtung mit einfachen Mitteln eine gezielte Belüftung unmittelbar unter dem Membranfilter zu erreichen, um nach Abnahme des Trichters das Beseitigen der Restflüssigkeit unter dem Membranfilter zu gewährleisten.

Gelöst wird diese Aufgabe durch eine Filtrationsvorrichtung für Vakuum-Membranfiltrationsanwendungen mit den Merkmalen des Anspruchs 1. Vorteilhafte und zweckmäßige Ausgestaltungen der erfindungsgemäßen Filtrationsbasis sind in den Unteransprüchen angegeben.

Die erfindungsgemäße Filtrationsvorrichtung ist für Vakuum-Membranfiltrationsanwendungen vorgesehen und umfasst eine Filtrationsbasis, einen lösbar auf der Filtrationsbasis montierten Aufgusstrichter und einem zwischen die Filtrationsbasis und den Aufgusstrichter eingeklemmten Membranfilter. Die Filtrationsbasis ist an eine Absaugvorrichtung anschließbar und umfasst einen Membranauflagebereich auf der Oberseite der Filtrationsbasis, der eine Auflagestruktur und eine die Auflagestruktur umgebende Abstützkontur für den im Membranauflagebereich platzierten Membranfilter aufweist. Die Abstützkontur grenzt außenseitig an eine Dichtfläche an, auf die der Rand des Membranfilters durch den auf die Filtrationsbasis aufgesetzten Aufgusstrichter gedrückt wird. Die Auflagestruktur und die Dichtfläche sind versetzt zueinander angeordnet, und ein Höhenunterschied zwischen der Auflagestruktur und der Dichtfläche wird durch die Abstützkontur überbrückt. Die Abstützkontur weist wenigstens eine Einkerbung auf, die in Strömungsverbindung mit der Unterseite des Membranauflagebereichs steht und so angeordnet ist, dass sie von dem im Membranauflagebereich platzierten Membranfilter selektiv abdeckbar ist. Die Einkerbung ist vom eingeklemmten Membranfilter abgedeckt, wenn der Aufgusstrichter auf der Filtrationsbasis montiert ist. Die Einkerbung ist nicht vom Membranfilter abgedeckt, wenn der Aufgusstrichter von der Filtrationsbasis abgenommen ist und sich der angesaugte benetzte

Membranfilter aufgrund eines von der Saugvorrichtung ausgeübten Unterdrucks nach oben wölbt.

Die Erfindung beruht auf der Erkenntnis, dass bei einer Vakuum-Membranfiltrationsvorrichtung für eine gezielte Belüftung der Unterseite des benetzten Membranfilters keine aufwendige Konstruktion mit einem eigens dafür vorgesehenen Ventil oder dergleichen, das separat geschaltet werden muss, erforderlich ist, wenn durch eine kleine, aber geschickte konstruktive Maßnahme das Verhalten des Membranfilters nach der Abnahme des Trichters ausgenutzt wird. Konkret macht sich die Erfindung zunutze, dass der weiterhin vom anliegenden Vakuum an die Oberseite der Filtrationsbasis angesaugte benetzte Membranfilter ohne den Trichter nicht mehr am Rand eingeklemmt ist, sodass sich der Rand nach oben wölben kann. Diese Verformung des Membranfilters kann bei der erfindungsgemäßen Filtrationsbasis dazu genutzt werden, eine gewollte Undichtigkeit im Zusammenspiel von Membranfilter und Filtrationsbasis hervorzurufen, indem eine speziell für die Belüftung vorgesehene Einkerbung, die vorher vom eingeklemmten Membranfilter abgedeckt war, freigegeben wird. Die freigegebene Einkerbung ermöglicht es der Umgebungsluft - unterstützt durch das weiterhin anliegende Vakuum - auf die Unterseite des Membranfilters zu strömen, sodass die unterhalb des Membranfilters noch vorhandene Restflüssigkeit abgesaugt und der Membranfilter leicht von der Filtrationsbasis abgenommen werden kann.

Vorzugsweise ist die Abstützkontur durch eine umlaufende Kante von der Auflagestruktur abgegrenzt, und die erfindungsgemäß vorgesehene Einkerbung unterbricht diese umlaufende Kante zwischen der Auflagestruktur und der Abstützkontur. Wenn der Rand des Membranfilters nicht mehr eingeklemmt ist und das Vakuum den mittleren Bereich des Membranfilters nach unten auf die Auflagestruktur der Filtrationsbasis zieht, wird der Membranfilter dabei gegen die Kante zwischen der Auflagestruktur und der Abstützkontur gedrückt. Der Membranfilter liegt dort also an sich noch dicht an der Filtrationsbasis an; die durch die Einkerbung geschaffene Unterbrechung sorgt jedoch für die gewollte Undichtigkeit, die einen Luftstrom unter den Membranfilter ermöglicht.

Die Einkerbung sollte zur Gewährleistung ihrer Funktion möglichst scharfkantig von ihrer Umgebung abgegrenzt sein. Ansonsten, d. h. wenn die Kanten der Einkerbung zu stark abgerundet wären, bestünde die Gefahr, dass sich der Membranfilter zu leicht an die abgerundeten Formen anlegt und in unerwünschter Weise auch hier eine dichte Verbindung mit der Filtrationsbasis eingeht. Die Form und die Abmessungen der Einkerbung sind in dieser Hinsicht auf die Steifheit handelsübliche Membranfilter abgestimmt, sodass ein unerwünschtes dichtes Anlegen eines solchen Membranfilters im benetzten Zustand bei einem typischen Absaugunterdruck ausgeschlossen ist.

Insbesondere dann, wenn die Filtrationsbasis als Kunststoff-Spritzgussteil gefertigt ist und die angestrebte scharfkantige Abgrenzung der Einkerbung aufgrund von Fertigungstoleranzen nicht ohne Weiteres gewährleistet ist, kann die Funktion der Einkerbung dadurch unterstützt werden, dass sich die Einkerbung in die Abstützkontur hinein erstreckt. Dank dieser Vergrößerung der Einkerbung steht dann ein erweitertes Ansaugfenster zur Verfügung.

Gemäß der Erfindung ist die Filtrationsbasis so gestaltet, dass die Abstützkontur außenseitig an eine Dichtfläche angrenzt, auf die der Rand des Membranfilters durch einen auf die Filtrationsbasis aufgesetzten Aufgusstrichter gedrückt werden kann. Bei einer solchen Gestaltung liegt der zwischen dem Trichter und der Dichtfläche der Filtrationsbasis eingeklemmte Membranfilter bei angelegtem Vakuum fest an der Abstützfläche an, sodass die Einkerbung abgedeckt ist. Wird dagegen nach der Filtration der Randbereich des Membranfilters nicht mehr vom Trichter auf die Dichtfläche gedrückt, reicht bereits ein leichtes Hochwölben des Membranfilterrands, um durch die in der angrenzenden Abstützkontur gebildete Einkerbung einen Strömungsweg unter den Membranfilter freizugeben.

Die Einkerbung sollte sich nicht von der Abstützkontur bis in die Dichtfläche hinein erstrecken, um die dichte Auflage des Membranfilterrands im eingeklemmten Zustand nicht zu beeinträchtigen.

Gemäß der Erfindung sind die Auflagestruktur und die Dichtfläche versetzt zueinander angeordnet, wobei ein Höhenunterschied zwischen der Auflagestruktur und der Dichtfläche durch die Abstützkontur überbrückt wird. Das bedeutet, dass der zunächst vom Trichter eingeklemmte Rand des Membranfilters höher liegt als der an den Auflagebereich angesaugte mittlere Bereich des Membranfilters. Diese Gestaltung unterstützt nach dem Abnehmen des Trichters das gewünschte Hochwölben des Membranfilterrands, um die Einkerbung freizugeben.

Typische Filtrationsbasen für Vakuum-Membranfiltrationsvorrichtungen haben einen Membranauflagebereich mit einem Außendurchmesser im Bereich von 15 bis 55 mm. Der hier bevorzugte Außendurchmesser liegt im Bereich von 25 bis 50 mm, insbesondere bevorzugt 45 bis 50 mm. Es erweist sich als besonders zweckmäßig, die wenigstens eine Einkerbung in einem Abstand von diesem Außendurchmesser im Bereich von 2 bis 10 mm, vorzugsweise von 3 bis 7 mm vorzusehen.

Bei einer bevorzugten Ausführungsform der erfindungsgemäßen Filtrationsbasis ist die Abstützkontur ringförmig, und es sind mehrere in Umfangsrichtung voneinander beabstandete Einkerbungen vorgesehen. Dank der Mehrzahl von Einkerbungen, die verteilt in der Abstützkontur angeordnet sind, ist eine zuverlässige Belüftung auch dann gewährleistet, wenn eine Einkerbung verblockt ist.

Im Hinblick auf eine bevorzugte symmetrische Gestaltung ist der Winkelabstand zwischen den Einkerbungen jeweils gleich groß ist und beträgt vorzugsweise 30°.

Die erfindungsgemäß in der Abstützkontur der Filtrationsbasis vorgesehene wenigstens eine Einkerbung kann grundsätzlich eine beliebig geformte Ausnehmung sein, die die gewünschte Strömungsverbindung zur Membranfilterunterseite herstellt. Es ist jedoch darauf zu achten, dass der Membranfilter bei abgenommenem Trichter nicht so in die erfindungsgemäße Einkerbung gesogen wird, dass er die Strömungsverbindung weiterhin blockiert. Die Einkerbung sollte entsprechend dieser Anforderung sehr klein bemessen sein, um sicherzustellen, dass der Membranfilter aufgrund seiner Eigensteifigkeit nicht signifikant in die Einkerbung eindringen kann. Es hat sich gezeigt, dass Einkerbungen mit einer Breite in Umfangsrichtung in einem Bereich von 0,1 bis 4 mm diese Anforderung erfüllen.

Natürlich sollte jede Einkerbung auch eine Mindesttiefe aufweisen, damit die Luft passieren kann. Eine bevorzugte Tiefe liegt im Bereich von 0,1 bis 1,5 mm.

Eine besonders vorteilhafte Gestaltung sieht vor, dass die wenigstens eine Einkerbung eine Verlängerung einer Abflussrille ist, die in der Auflagestruktur gebildet ist. Ziel ist es, nach einer Filtration möglichst viel von der noch in der Auflagestruktur befindlichen Restflüssigkeit abzusaugen. In der Auflagestruktur der Filtrationsbasis sind Abflussrillen dafür vorgesehen, die während der Filtration durch den Membranfilter gesaugte Flüssigkeit zu sammeln und zum Abfluss der Filtrationsbasis fließen zu lassen. Wenn nun nach der Filtration dank der Einkerbungen Luft unter den Membranfilter gesaugt und gezielt in eine Abflussrille geleitet wird, ist die Mitnahme von Restflüssigkeit besonders effektiv.

Die erfindungsgemäße Filtrationsbasis kann als Mehrweg- oder Einwegkomponente ausgelegt sein. In ersterem Fall ist sie vorzugsweise aus Edelstahl, in letzterem Fall vorzugsweise aus einem sterilisierbaren Kunststoff gebildet. Dies eröffnet die Möglichkeit, die Filtrationsbasis - ggf. zusammen mit weiteren Komponenten der Filtrationsvorrichtung - vorzusterilisieren. Damit ist die Filtrationsbasis nach der Auslieferung sofort einsatzbereit.

Die Erfindung schafft auch eine Filtrationsvorrichtung mit einer erfindungsgemäßen Filtrationsbasis, einem lösbar auf der Filtrationsbasis montierten Aufgusstrichter und einem zwischen die Filtrationsbasis und den Aufgusstrichter eingeklemmten Membranfilter.

Zum Einklemmen des Membranfilters kann auf Seiten der Filtrationsbasis eine die Abstützkontur umgebende Dichtfläche und auf Seiten des Trichters eine passende Auswölbung vorgesehen sein, die den Rand des Membranfilters auf die Dichtfläche drückt. Grundsätzlich können Dichtfläche und Auswölbung auch vertauscht sein, d. h. die Dichtfläche kann am Trichter und die Auswölbung an der Filtrationsbasis gebildet sein. Dank der dadurch erreichten Abdichtung ist bei aufgesetztem Filter eine Filtration auch ohne Ansaugen von Luft möglich.

Für die typischen Anwendungsfälle ist die Filtrationsbasis aber an eine Absaugvorrichtung angeschlossen, mit der ein Vakuum (Unterdruck) unter der Filtrationsbasis erzeugt wird, um die Filtration zu beschleunigen oder überhaupt erst zu ermöglichen.

Wie bereits erläutert sollte die wenigstens eine Einkerbung so klein bemessen sein, dass sich bei einem für einen Filtrationsvorgang von der Absaugvorrichtung erzeugten Unterdruck der Membranfilter aufgrund seiner Steifheit nicht dicht an die Kontur der Einkerbung anlegt.

Im Hinblick auf eine bevorzugte Einweg-Verwendung sind die Filtrationsbasis und/oder der Trichter aus einem (vor-)sterilisierbaren Kunststoff gefertigt. Falls eine Mehrfach-Verwendung vorgesehen ist, sind diese Komponenten typischerweise aus Edelstahl oder Aluminium gefertigt. In jedem Fall ist die erfindungsgemäße Filtrationsvorrichtung auf einer Unterdruck-basierten Absaugvorrichtung (Absaugleiste) montierbar. Die Absaugvorrichtung kann wiederum aus sterilisierbarem Edelstahl oder Aluminium (Mehrweg-Verwendung) oder aus sterilisierbarem Kunststoff (Einweg-Verwendung) gefertigt sein.

Weitere Merkmale und Vorteile der Erfindung ergeben sich aus der nachfolgenden Beschreibung und aus den beigefügten Zeichnungen, auf die Bezug genommen wird. In den Zeichnungen zeigen:
- Figur 1 eine Seitenansicht einer erfindungsgemäßen Filtrationsvorrichtung;
- Figur 2 eine perspektivische Ansicht der erfindungsgemäßen Filtrationsbasis der Filtrationsvorrichtung aus Figur 1;
- Figur 3 eine seitliche Schnittansicht der Filtrationsbasis aus Figur 2;
- Figur 4 eine perspektivische Ansicht eines Randbereichs der Filtrationsbasis aus Figur 3;
- Figur 5 eine Draufsicht auf einen Randbereich der Filtrationsbasis aus Figur 3; und
- Figur 6 eine geschnittene Detailansicht des Klemmbereiches der Filtrationsvorrichtung aus Figur 1.

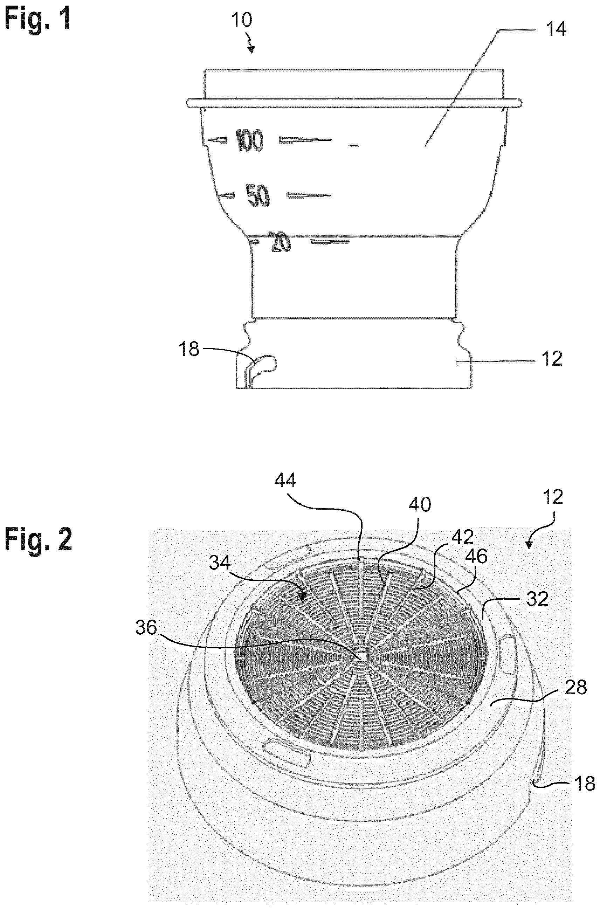

In Figur 1 ist eine Filtrationsvorrichtung 10 dargestellt, die für Vakuum-Membranfiltrationsanwendungen vorgesehen ist. Auf einer Filtrationsbasis 12 ist ein Aufgusstrichter 14 lösbar montiert. Filtrationsbasis 12 und Trichter 14 sind typischerweise mehrfach verwendbare Edelstahleinheiten. Im vorliegenden Fall sind aber Bauformen aus Kunststoff bevorzugt, die vorsterilisiert werden können und für die Einwegverwendung vorgesehen sind.

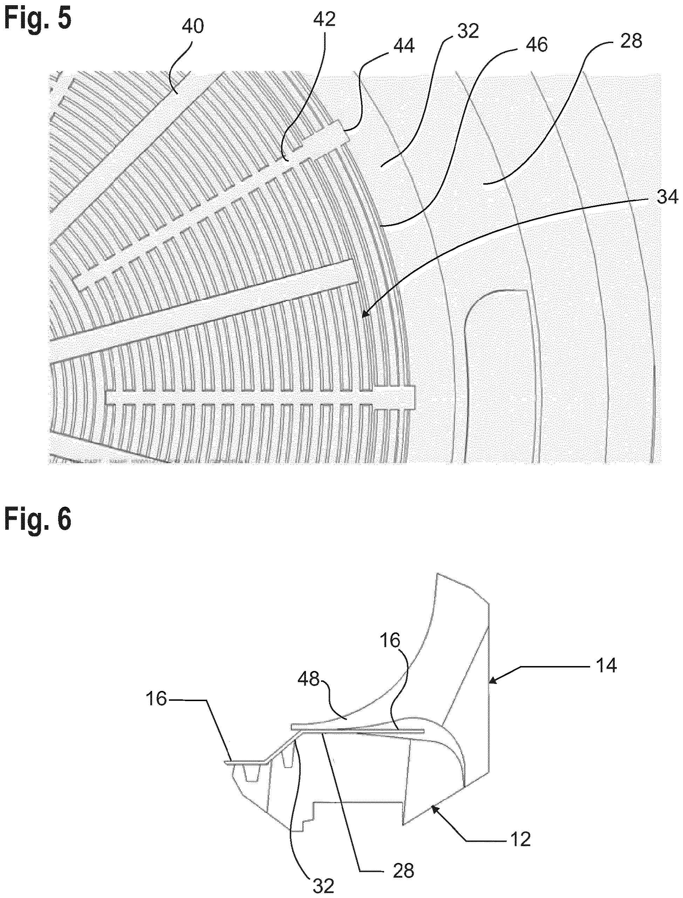

Zwischen der Filtrationsbasis 12 und dem Trichter 14 ist ein Membranfilter 16 (siehe Schnittansicht in Figur 6) eingeklemmt.

Die Filtrationsvorrichtung 10 kann auf einer Absaugleiste oder einer anderen Absaugvorrichtung aus Edelstahl (Mehrwegverwendung) oder Kunststoff (Einwegverwendung) montiert werden. Hierfür ist in der Filtrationsbasis 12 eine Aussparung 18 vorgesehen, die zusammen mit einem passenden Vorsprung an einem Anschlussstück der Absaugvorrichtung einen Bajonettverschluss bildet. Die Fixierung ist auch über eine Steckverbindung realisierbar, bei der keine Drehbewegung wie beim Bajonettverschluss notwendig ist.

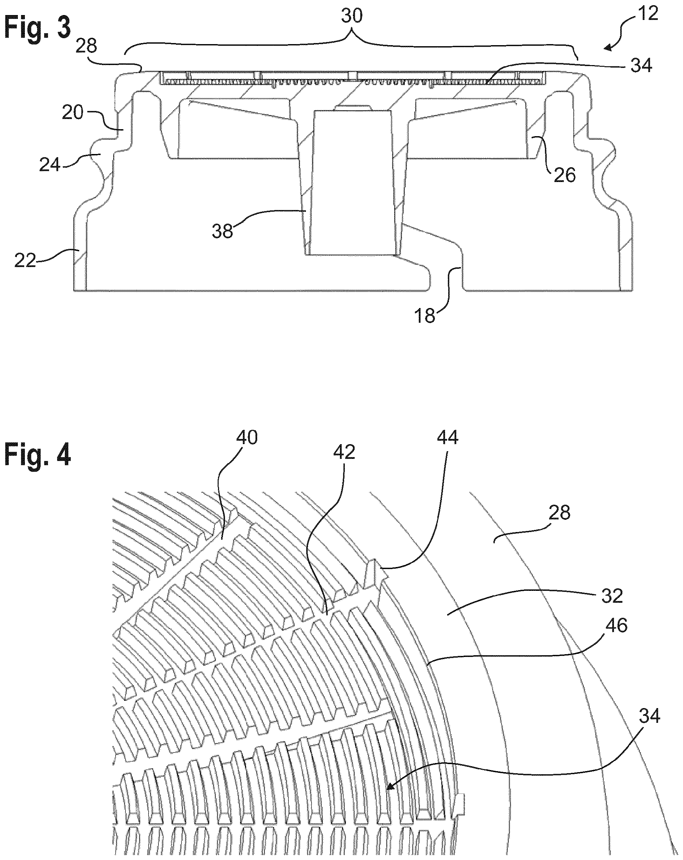

Die Filtrationsbasis 12 ist in den Figuren 2 und 3 separat gezeigt. Die grundsätzlich kreisrunde Filtrationsbasis 12 weist einen oberen ersten zylindrischen Abschnitt 20 mit einem bevorzugten Außendurchmesser von 49 mm und unterhalb der Schulter einen unteren zweiten zylindrischen Abschnitt 22 auf. Auf den ersten zylindrischen Abschnitt 20 wird der Trichter 14 aufgeschoben, wobei eine umlaufende Schulter 24 als Auflagefläche für die Stirnseite des Trichters 14 dient. Der untere zylindrische Abschnitt 22 wird mit dem Anschlussstück der Absaugvorrichtung über den Bajonett-Verschluss oder die Steckverbindung so verbunden, dass das Anschlussstück gegen eine Dichtkontur 26 und/oder eine später noch genauer erläuterte Begrenzungswand 38 der Filtrationsbasis 12 gedrückt wird. Dadurch ist ein dichter Anschluss an ein von der Absaugvorrichtung bereitgestelltes Vakuum (Unterdruck) sichergestellt.

Wie in den Figuren 3 bis 5 zu erkennen ist, weist die Filtrationsbasis 12 auf ihrer Oberseite einen Membranauflagebereich 30 auf, der unterteilt ist in eine an den oberen zylindrischen Abschnitt 20 angrenzende ringförmige Dichtfläche 28, eine an die Dichtfläche 28 angrenzende Abstützkontur 32 und eine von der Abstützkontur 32 umgebene profilierte Auflagestruktur 34. Sowohl die Dichtfläche 28 als auch die profilierte Auflagestruktur 34 erstrecken sich im Wesentlichen jeweils in einer zur Mittelachse der Filtrationsbasis 12 senkrechten Ebene. Die profilierte Auflagestruktur 34 ist aber relativ zur Dichtfläche 28 nach unten versetzt, wobei sich zwischen der profilierten Auflagestruktur 34 und der erhöhten ringförmigen Dichtfläche 28 die Abstützkontur 32 erstreckt.

Die Abstützkontur 32 stellt einerseits eine definierte Abgrenzung zur ringförmigen Dichtfläche 28 dar, andererseits kann sie als Fortsetzung der profilierten Auflagestruktur 34 oder als eine andere profilierte Struktur (z. B. mit Stufen) oder als ebene Fläche ausgebildet sein. In jedem Fall überbrückt die Abstützkontur 32 den axialen Höhenunterschied zwischen der profilierten Auflagestruktur 34 und der Dichtfläche 28.

Der Membranauflagebereich 30 ist in der Mitte durch einen zentralen Abfluss 36 unterbrochen, der eine Strömungsverbindung zum Bereich unter dem Membranauflagebereich 30 und damit zur Absaugvorrichtung herstellt. Die nach unten ragende Begrenzungswand 38 des Abflusses 36 kann zusätzlich oder alternativ zur Dichtkontur 26 als Dichtfläche 28 für den Anschluss an die Absaugvorrichtung genutzt werden.

Die profilierte Auflagestruktur 34 des Membranauflagebereichs 30 weist eine Vielzahl konzentrischer Rillen auf, es sind aber auch andere Strukturen möglich. Mehrere Abflussrillen 40, 42 sorgen dafür, dass die durch den Membranfilter 16 durchgetretene Flüssigkeit zum Abfluss 36 gelangen kann. Die Abflussrillen 40, 42 sind in Umfangsrichtung gleichmäßig verteilt und verlaufen gerade in radialer Richtung. Im dargestellten Ausführungsbeispiel sind vier große Abflussrillen 40 jeweils im 90°-Abstand und 12 kleine Abflussrillen 42 jeweils im 30°-Abstand vorgesehen.

Wie in den Detailansichten der Figuren 4 und 5 erkennbar ist, erstrecken sich die großen Abflussrillen 40 nach außen nicht ganz bis zur schrägen Abstützkontur 32, während die kleinen Abflussrillen 42 jeweils in eine Einkerbung 44 der Abstützkontur 32 münden. Die Einkerbung 44 unterbricht eine umlaufende Kante 46 zwischen der Abstützkontur 32 und der profilierten Auflagestruktur 34. Somit ist die den Membranfilter 16 abstützende Kontur 32 in Umfangsrichtung in einem Winkelabstand von jeweils 30° durch eine Einkerbung 44 unterbrochen.

Die durch scharfe Kanten abgegrenzten Einkerbungen 44 sind jeweils 1,0 mm breit und 1,4 mm tief und befinden sich in einem Bereich, der 4,6 mm bis 5,6 mm vom Außenumfang des oberen zylindrischen Abschnitts 20 nach innen beabstandet ist.

Im dargestellten Ausführungsbeispiel erstrecken sich die Einkerbungen 44 radial in die Abstützkontur 32 hinein (siehe Figuren 4 und 5). Je nach verwendetem Material und Präzision des Fertigungsverfahrens ist diese radiale Erstreckung für die weiter unten erläuterte Funktion der Einkerbungen 44 nicht zwingend notwendig, empfiehlt sich jedoch insbesondere dann, wenn die Filtrationsbasis 12 als Kunststoff-Spritzgussteil gefertigt wird.

Nachfolgend werden der Filtrationsbetrieb der Filtrationsvorrichtung 10 und die anschließende Belüftung beschrieben.

Vor dem Filtrationsbetrieb wird ein Membranfilter 16 auf die Oberseite der Filtrationsbasis 12 gelegt und der Aufgusstrichter 14 auf die Filtrationsbasis 12 aufgesetzt. Dabei drückt eine nach innen gerichtete umlaufende Auswölbung 48 des Trichters 14 den Außenrand des Membranfilters 16 fest auf die Dichtfläche 28 der Filtrationsbasis 12, wie in der Detailansicht der Figur 6 zu sehen ist.

Obwohl grundsätzlich auch eine Filtration mit Überdruck auf Seiten des Trichters 14 möglich ist, ist die Filtrationsvorrichtung 10 in der Regel an eine Absaugvorrichtung angeschlossen, die unterhalb der Filtrationsbasis 12 ein Vakuum erzeugt, um die im Trichter 14 enthaltene Flüssigkeit durch den zwischen Filtrationsbasis 12 und Trichter 14 eingeklemmten Membranfilter 16 anzusaugen. Dabei wird der Membranfilter 16 an die profilierte Auflagestruktur 34 des Membranauflagebereichs 30 gezogen und legt sich an die Abstützkontur 32 an. Die durch den Membranfilter 16 angesaugte Flüssigkeit wird durch die profilierte Auflagestruktur 34 und die Abflussrillen 40, 42 zum Abfluss 36 geleitet.

Nach der Filtration wird der Trichter 14 abgenommen, während das Vakuum immer noch anliegt. Da der Membranfilter 16 nun nicht mehr vom Trichter 14 gegen die Dichtfläche 28 gedrückt wird, kann sich der Rand des weiterhin vom Vakuum an die Filtrationsbasis 12 angezogenen benetzten Membranfilters 16 nach oben wölben, wie in den Detailansichten der Figuren 5 und 6 dargestellt. Dabei liegt der Membranfilter 16 aber immer noch fest an der schrägen Abstützkontur 32 und an der Kante 46 zwischen der Abstützkontur 32 und der Dichtfläche 28 an, sodass in diesen Anlagebereichen keine Umgebungsluft unter den Membranfilter 16 gelangen kann.

Da jedoch die Kante 46 zwischen der Abstützkontur 32 und der profilierte Auflagestruktur 34 durch die Einkerbungen 44 unterbrochen ist, wird an diesen Stellen aufgrund des anliegenden Vakuums Luft durch die Einkerbungen 44 in die kleinen Abflussrillen 42 und damit in den Bereich unter dem Membranfilter 16 gesogen.

Wie bereits erläutert sind die Einkerbungen 44 sehr klein bemessen (Breite in Umlaufrichtung und Tiefe) und durch scharfe Kanten abgegrenzt. Deshalb wird bei einem für einen Filtrationsvorgang typischen Unterdruck ein handelsüblicher Membranfilter 16 im benetzten Zustand aufgrund seiner Steifheit nicht in die Einkerbungen 44 hineingezogen und behindert nicht das Einsaugen der Luft oder den Abtransport der Restflüssigkeit. Wenn sich die Einkerbungen 44 wie beim dargestellten Ausführungsbeispiel radial in die Abstützkontur 32 hinein erstrecken, ist das Ansaugen von Luft durch die Einkerbungen 44 auch dann gewährleistet, wenn die Kanten der Einkerbungen 44 z. B. aufgrund von Fertigungstoleranzen weniger scharfkantig sind.

Dank der eingesaugten Luft kann das unter dem Membranfilter 16 befindliche Flüssigkeitsvolumen durch den Abfluss 36 abfließen. Außerdem ist dank der Belüftung ein Abheben des Membranfilters 16 von der Filtrationsbasis 12 trotz des anliegenden Vakuums relativ leicht möglich.

### Bezugszeichenliste

- 10: Filtrationsvorrichtung
- 12: Filtrationsbasis
- 14: Aufgusstrichter
- 16: Membranfilter
- 18: Aussparung
- 20: erster zylindrischer Abschnitt
- 22: zweiter zylindrischer Abschnitt
- 24: Schulter
- 26: Dichtkontur
- 28: Dichtfläche
- 30: Membranauflagebereich
- 32: Abstützkontur
- 34: profilierte Auflagestruktur
- 36: Abfluss
- 38: Abflussbegrenzungswand
- 40: große Abflussrillen
- 42: kleine Abflussrillen
- 44: Einkerbung
- 46: Kante
- 48: Auswölbung

## Patentansprüche

1. Filtrationsvorrichtung (10) für Vakuum-Membranfiltrationsanwendungen, mit einer Filtrationsbasis (12), einem lösbar auf der Filtrationsbasis (12) montierten Aufgusstrichter (14) und einem zwischen die Filtrationsbasis (12) und den Aufgusstrichter (14) eingeklemmten Membranfilter (16), wobei die Filtrationsbasis (12) an eine Absaugvorrichtung anschließbar ist und einen Membranauflagebereich (30) auf der Oberseite der Filtrationsbasis (12) umfasst, der eine Auflagestruktur (34) und eine die Auflagestruktur (34) umgebende Abstützkontur (32) für den im Membranauflagebereich (30) platzierten Membranfilter (16) aufweist, wobei die Abstützkontur (32) außenseitig an eine Dichtfläche (28) angrenzt, auf die der Rand des Membranfilters (16) durch den auf die Filtrationsbasis (12) aufgesetzten Aufgusstrichter (14) gedrückt wird, wobei die Auflagestruktur (34) und die Dichtfläche (28) versetzt zueinander angeordnet sind und ein Höhenunterschied zwischen der Auflagestruktur (34) und der Dichtfläche (28) durch die Abstützkontur (32) überbrückt wird, wobei die Abstützkontur (32) wenigstens eine Einkerbung (44) aufweist, die in Strömungsverbindung mit der Unterseite des Membranauflagebereichs (30) steht und so angeordnet ist, dass sie von dem im Membranauflagebereich (30) platzierten Membranfilter (16) selektiv abdeckbar ist, wobei die Einkerbung (44) vom eingeklemmten Membranfilter (16) abgedeckt ist, wenn der Aufgusstrichter (14) auf der Filtrationsbasis (12) montiert ist, und die Einkerbung (44) nicht vom Membranfilter (16) abgedeckt ist, wenn der Aufgusstrichter (14) von der Filtrationsbasis (12) abgenommen ist und sich der angesaugte benetzte Membranfilter (16) aufgrund eines von der Saugvorrichtung ausgeübter Unterdrucks nach oben wölbt.

2. Filtrationsvorrichtung (10) nach Anspruch 1, **dadurch gekennzeichnet, dass** die Einkerbung (44) eine umlaufende Kante (46) zwischen der Auflagestruktur (34) und der Abstützkontur (32) unterbricht.

3. Filtrationsvorrichtung (10) nach Anspruch 1 oder 2, **dadurch gekennzeichnet, dass** die Einkerbung (44) scharfkantig abgegrenzt ist.

4. Filtrationsvorrichtung (10) nach einem der vorhergehenden Ansprüche, **dadurch gekennzeichnet, dass** sich die Einkerbung (44) in die Abstützkontur (32) erstreckt.

5. Filtrationsvorrichtung (10) nach einem der vorhergehenden Ansprüche, **dadurch gekennzeichnet, dass** sich die Einkerbung (44) nicht in die Dichtfläche (28) erstreckt.

6. Filtrationsvorrichtung (10) nach einem der vorhergehenden Ansprüche, **dadurch gekennzeichnet, dass** der Membranauflagebereich (30) einen Außendurchmesser im Bereich von 15 bis 55 mm, vorzugsweise 25 bis 50 mm, insbesondere bevorzugt 45 bis 50 mm hat und die wenigstens eine Einkerbung (44) in einem Abstand vom Außendurchmesser im Bereich von 2 bis 10 mm, vorzugsweise von 3 bis 7 mm gebildet ist.

7. Filtrationsvorrichtung (10) nach einem der vorhergehenden Ansprüche, **dadurch gekennzeichnet, dass** die Abstützkontur (32) ringförmig ist und mehrere in Umfangsrichtung voneinander beabstandete Einkerbungen (44) vorgesehen sind.

8. Filtrationsvorrichtung (10) nach Anspruch 7, **dadurch gekennzeichnet, dass** der Winkelabstand zwischen den Einkerbungen (44) jeweils gleich groß ist und vorzugsweise 30° beträgt.

9. Filtrationsvorrichtung (10) nach Anspruch 7 oder 8, **dadurch gekennzeichnet, dass** die Einkerbungen (44) eine Breite in Umfangsrichtung im Bereich von 0,1 bis 4 mm aufweisen.

10. Filtrationsvorrichtung (10) nach einem der Ansprüche 7 bis 9, **dadurch gekennzeichnet, dass** die Einkerbungen (44) eine Tiefe im Bereich von 0,1 bis 1,5 mm aufweisen.

11. Filtrationsvorrichtung (10) nach einem der vorhergehenden Ansprüche, **dadurch gekennzeichnet, dass** die wenigstens eine Einkerbung (44) eine Verlängerung einer Abflussrille (42) ist, die in der Auflagestruktur (34) gebildet ist.

12. Filtrationsvorrichtung (10) nach einem der vorhergehenden Ansprüche, **dadurch gekennzeichnet, dass** die Filtrationsbasis aus einem sterilisierbaren Kunststoff besteht.

13. Filtrationsvorrichtung (10) nach einem der vorhergehenden Ansprüche, **dadurch gekennzeichnet, dass** der Membranfilter (16) zwischen einer die Abstützkontur (32) umgebenden Dichtfläche (28) und einer Auswölbung (48) des Aufgusstrichters (14) eingeklemmt ist.

14. Filtrationsvorrichtung (10) nach einem der vorhergehenden Ansprüche, **dadurch gekennzeichnet, dass** die wenigstens eine Einkerbung (44) so klein bemessen ist, dass sich bei einem für einen Filtrationsvorgang von der Absaugvorrichtung erzeugten Unterdruck der Membranfilter (16) aufgrund seiner Steifheit nicht dicht an die Kontur der Einkerbung (44) anlegt.

15. Filtrationsvorrichtung (10) nach einem der einem der vorhergehenden Ansprüche, **dadurch gekennzeichnet, dass** der Aufgusstrichter (14) und/oder die Absaugvorrichtung aus einem sterilisierbaren Kunststoff bestehen.

## Claims

1. A filtration device (10) for vacuum membrane filtration applications, having a filtration base (12), a pour funnel (14) detachably mounted on the filtration base (12) and a membrane filter (16) clamped between the filtration base (12) and the pour funnel (14), wherein the filtration base (12) can be connected to a suction device and comprises a membrane rest region (3) on the top side of the filtration base (12), which has a rest structure (34) and a support contour (32) surrounding the rest structure (34) for the membrane filter (16) placed in the membrane rest region(30), wherein the support contour (32) adjoins on its outside a sealing surface (28), onto which the rim of the membrane filter (16) is pressed through the pour funnel (14) placed upon the filtration base (12), wherein the rest structure (34) and the sealing surface (28) are arranged offset to each other and a height difference between the rest structure (34) and the sealing surface (28) is bridged by the support contour (32), wherein the support contour (32) has at least one notch (44), which is in flow connection to the underside of the membrane rest region (30) and is arranged such that it can be selectively covered by the membrane filter (16) placed in the membrane rest region (30), wherein the notch (44) is covered by the clamped membrane filter (16) when the pour funnel (14) is mounted on the filtration base (12), and the notch (44) is not covered by the membrane filter (16) when the pour funnel (14) is removed from the filtration base (12) and the aspirated moistened membrane filter (16) curves upward due to a negative pressure exerted by the suction device.

2. The filtration device (10) according to claim 1, **characterized in that** the notch (44) interrupts a circumferential edge (46) between the rest structure (34) and the support contour (32).

3. The filtration device (10) according to claim 1 or 2, **characterized in that** the notch (44) is delimited in a sharpedged manner.

4. The filtration device (10) according to any of the foregoing claims, **characterized in that** the notch (44) extends into the support contour (32).

5. The filtration device (10) according to any of the foregoing claims, **characterized in that** the notch (44) does not extend into the sealing surface (28).

6. The filtration device (10) according to any of the foregoing claims, **characterized in that** the membrane rest region (30) has an outer diameter in the range of 15 to 55 mm, preferably 25 to 50 mm, in particular preferably 45 to 50 mm and the at least one notch (44) is formed at a distance from the outer diameter in the range of 2 to 10 mm, preferably from 3 to 7 mm.

7. The filtration device (10) according to any of the foregoing claims, **characterized in that** the support contour (32) is annular and a plurality of notches (44) spaced apart from one another in circumferential direction are provided.

8. The filtration device (10) according to claim 7, **characterized in that** the angular distance between the notches (44) is respectively the same and preferably 30°.

9. The filtration device (10) according to claim 7 or 8, **characterized in that** the notches (44) have a width in circumferential direction in the range of 0.1 to 4 mm.

10. The filtration device (10) according to any of claims 7 to 9, **characterized in that** the notches (44) have a depth in the range of 0.1 to 1.5 mm.

11. The filtration device (10) according to any of the foregoing claims, **characterized in that** the at least one notch (44) is a prolongation of a discharge groove (42), which is formed in the rest structure (34).

12. The filtration device (10) according to any of the foregoing claims, **characterized in that** the filtration base consists of a sterilizable plastic.

13. The filtration device (10) according to any of the foregoing claims, **characterized in that** the membrane filter (16) is clamped wedged between a sealing surface (28) surrounding the support contour (32) and a bulge (48) of the pour funnel (14).

14. The filtration device (10) according to any of the foregoing claims, **characterized in that** the at least one notch (44) is dimensioned so small that in the event of a negative pressure generated for a filtration process by the suction device the membrane filter (16) does not fit tightly on the contour of the notch (44) due to its stiffness.

15. The filtration device (10) according to any of the foregoing claims, **characterized in that** the pour funnel (14) and/or the suction device consist of a sterilizable plastic.

## Revendications

1. Dispositif de filtration (10) pour des utilisations de filtration sur membrane sous vide, avec une base de filtration (12), un entonnoir à infusion (14) monté de façon amovible sur la base de filtration (12) et un filtre à membrane (16) enserré entre la base de filtration (12) et l'entonnoir à infusion (14), dans lequel la base de filtration (12) peut être raccordée à un dispositif d'aspiration et comprend, sur le côté supérieur de la base de filtration (12), une région de support de membrane (30) qui présente une structure de support (34) et un contour d'appui (32) entourant la structure de support (34) pour le filtre à membrane (16) placé dans la région de support de membrane (30), dans lequel le contour d'appui (32) est contigu sur le côté extérieur avec une surface d'étanchéité (28) sur laquelle le bord du filtre à membrane (16) est poussé par l'entonnoir à infusion (14) posé sur la base de filtration (12), dans lequel la structure de support (34) et la surface d'étanchéité (28) sont agencées en décalage l'une par rapport à l'autre et une différence de hauteur entre la structure de support (34) et la surface d'étanchéité (28) est comblée par le contour d'appui (32), dans lequel le contour d'appui (32) présente au moins une encoche (44) qui se trouve en liaison d'écoulement avec le côté inférieur de la région de support de membrane (30) et est agencé de sorte qu'il puisse être sélectivement recouvert par le filtre à membrane (16) placé dans la région de support de membrane (30), dans lequel l'encoche (44) est recouverte par le filtre à membrane (16) enserré lorsque l'entonnoir à infusion (14) est monté sur la base de filtration (12), et l'encoche (44) n'est pas recouverte par le filtre à membrane (16) lorsque l'entonnoir à infusion (14) est retiré de la base de filtration (12) et que le filtre à membrane (16) mouillé et aspiré se bombe vers le haut du fait d'une dépression exercée par le dispositif d'aspiration.

2. Dispositif de filtration (10) selon la revendication 1, **caractérisé en ce que** l'encoche (44) interrompt un rebord (46) périphérique entre la structure de support (34) et le contour d'appui (32).

3. Dispositif de filtration (10) selon la revendication 1 ou 2, **caractérisé en ce que** l'encoche (44) est délimitée par arête vive.

4. Dispositif de filtration (10) selon l'une des revendications précédentes, **caractérisé en ce que** l'encoche (44) s'étend jusque dans le contour d'appui (32).

5. Dispositif de filtration (10) selon l'une des revendications précédentes, **caractérisé en ce que** l'encoche (44) ne s'étend pas jusque dans la surface d'étanchéité (28).

6. Dispositif de filtration (10) selon l'une des revendications précédentes, **caractérisé en ce que** la région de support de membrane (30) a un diamètre extérieur dans la plage de 15 à 55 mm, de préférence de 25 à 50 mm, en particulier de préférence de 45 à 50 mm, et la au moins une encoche (44) est formée à une distance du diamètre extérieur dans la plage de 2 à 10 mm, de préférence de 3 à 7 mm.

7. Dispositif de filtration (10) selon l'une des revendications précédentes, **caractérisé en ce que** le contour d'appui (32) est annulaire et que sont prévues plusieurs encoches (44) à distance les unes des autres dans la direction périphérique.

8. Dispositif de filtration (10) selon la revendication 7, **caractérisé en ce que** la distance angulaire entre les encoches (44) est respectivement de même taille et s'élève de préférence à 30°.

9. Dispositif de filtration (10) selon la revendication 7 ou 8, **caractérisé en ce que** les encoches (44) présentent dans la direction périphérique une largeur dans la plage de 0,1 à 4 mm.

10. Dispositif de filtration (10) selon l'une des revendications 7 à 9, **caractérisé en ce que** les encoches (44) présentent une profondeur dans la plage de 0,1 à 1,5 mm.

11. Dispositif de filtration (10) selon l'une des revendications précédentes, **caractérisé en ce que** la au moins une encoche (44) est un prolongement d'une rainure d'évacuation (42) qui est formée dans la structure de support (34).

12. Dispositif de filtration (10) selon l'une des revendications précédentes, **caractérisé en ce que** la base de filtration est composée d'une matière plastique stérilisable.

13. Dispositif de filtration (10) selon l'une des revendications précédentes, **caractérisé en ce que** le filtre à membrane (16) est enserré entre une surface d'étanchéité (28) entourant le contour d'appui (32) et un bombement vers l'extérieur (48) de l'entonnoir à infusion (14).

14. Dispositif de filtration (10) selon l'une des revendications précédentes, **caractérisé en ce que** la au moins une encoche (44) est dimensionnée suffisamment petite pour que, lors d'une dépression produite par le dispositif d'aspiration pour un processus de filtration, le filtre à membrane (16) ne s'applique pas de façon étanche contre le contour de l'encoche (44) du fait de sa rigidité.

15. Dispositif de filtration (10) selon l'une des revendications précédentes, **caractérisé en ce que** l'entonnoir à infusion (14) et/ou le dispositif d'aspiration sont composés d'une matière plastique stérilisable.
